# EUROPÄISCHE PATENTSCHRIFT

(11) **EP 0 831 095 B1**
(45) Veröffentlichungstag und Bekanntmachung des Hinweises auf die Patenterteilung: **31.10.2001**
(21) Anmeldenummer: 97810645.8
(22) Anmeldetag: 10.09.1997
(51) Int. Cl.: C07D 487/04

(54) **Verfahren zur Herstellung von Diketopyrrolopyrrol-Carbonsäuren, deren Estern und Amiden**
Process for the preparation of dicetopyrrolopyrrole carboxylic acids, their esters and amides
Procédé pour la préparation d'acides, carboxyliques de dicétopyrrolopyrroles, leurs esters et amides

(30) Priorität: 19.09.1996 CH 229896
(43) Veröffentlichungstag der Anmeldung: 25.03.1998
(73) Patentinhaber: Ciba Specialty Chemicals Holding Inc., 4057 Basel (CH)
(72) Erfinder: Lamatsch, Bernd, 1723 Marly (CH); Wallquist, Olof, 1723 Marly (CH)

(56) Entgegenhaltungen:
- US-A- 4 579 949

## Beschreibung

Die vorliegende Erfindung betrifft die Herstellung von Diketopyrrolopyrrol-Carbonsäuren, deren Estern und Amiden ausgehend von Diketopyrrolopyrrol-Halogenverbindungen.

In den US-Patenten 4 415 685, 4 579 949 und 4 791 204 sind Diketopyrrolopyrrol-Carbonsäuren, deren Ester und Amide beschrieben, die durch Umsetzung von Bernsteinsäuredialkylestern mit im allgemeinen schwer zugänglichen, durch eine entsprechende Carbonsäure-, -ester- oder -amidgruppe substituierten, Benzonitrilen hergestellt werden. Diketopyrrolopyrrol-Carbonamide sind auch in US-Patent 5 476 886 beschrieben. Sie werden aus entsprechenden Diketopyrrolopyrrol-Cyanderivaten durch Versetzung mit konzentrierter Schwefelsäure erhalten.

Aufgabe der vorliegenden Erfindung war es daher ein verbessertes Verfahren zur Herstellung von Diketopyrrolopyrrol-Carbonsäuren, deren Estern und Amiden, ausgehend von Diketopyrrolopyrrol-Halogenverbindungen, zur Verfügung zu stellen. Insbesondere sollten dabei die erfindungsgemäß hergestellten Verbindungen in höheren Ausbeuten, in einer höheren chemischen Reinheit herstellbar sein und eine ausgezeichnete Farbtonreinheit aufweisen.

Demgemäß wurde ein verbessertes Verfahren zur Herstellung von Diketopyrrolopyrrolen der Formel I 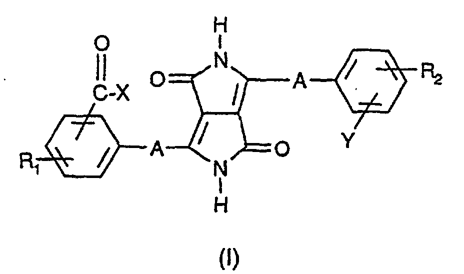 durch Umsetzung eines Diketopyrrolopyrrols der Formel II 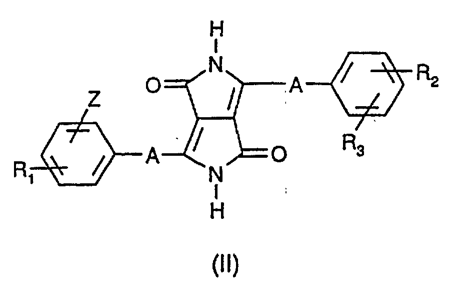 mit
a) Ameisensäure oder
b) einer Verbindung der Formel III

   X'-H (III)

   oder einem Alkalimetall- oder einem Erdalkalimetallformiat
   zusammen mit Kohlenmonoxid
   in Gegenwart eines Katalysators gefunden,
   wobei in den Formeln I, II und III
   A eine direkte Bindung oder eine Gruppe 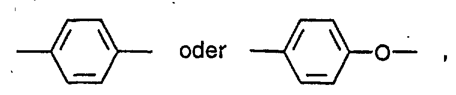 wobei das Phenylen an das Pyrrolopyrrol gebunden ist, bedeutet,
   R₁, R₂ und R₃ unabhängig voneinander Wasserstoff, Halogen, C₁-C₆-Alkyl, C₅-C₆-Cycloalkyl, C₁-C₆-Alkoxy, C₁-C₆-Alkylamino, C₁-C₆-Alkylmercapto, -CF₃, -CN oder -NO₂ bedeuten,
   X X' oder OH ist,
   X' -OR₄ oder -N(R₅)(R₆) bedeutet, worin
   R₄ C₁-C₁₈-Alkyl, C₅-C₆-Cycloalkyl, unsubstituiertes oder durch Halogen, C₁-C₆-Alkyl, C₁-C₆-Alkoxy, C₁-C₆-Alkylamino, -CN oder -NO₂ substituiertes Phenyl oder Naphthyl ist,
   R₅ und R₆ unabhängig voneinander Wasserstoff, C₁-C₁₈-Alkyl, C₅-C₆-Cycloalkyl, unsubstituiertes oder durch Halogen, C₁-C₆-Alkyl, C₁-C₆-Alkoxy, C₄-C₆-Alkylamino, -CN oder -NO₂ substituiertes Phenyl oder Naphthyl oder eine Gruppe -(CH₂)ₙ-SO₃R₇ bedeuten, worin R₇ Wasserstoff, Kalium oder Natrium und n eine ganze Zahl zwischen 2 und 6 sind, oder R₅ und R₆ zusammen mit dem N-Atom, an das sie gebunden sind, einen unsubstituierten oder durch C₁-C₆-Alkyl oder Phenyl substituierten 5- oder 6-gliedrigen heterocyclischen Rest ausgewählt aus der Gruppe Pyrrolidinyl, Piperidyl, Pyrrolyl, Triazolyl, Imidazolyl, Pyrazolyl,
   Piperazinyl, Morpholinyl, Thiomorpholinyl, Phthalimidyl, Carbazolyl, Indolyl, Indazolyl, Benzimidazolyl und Tetrahydrochinolinyl bilden,
   Y R₃ oder 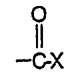 bedeutet, und
   Z Halogen ist.

Bedeuten etwaige Substitutenten Halogen, dann handelt es sich in der Regel um lod, Fluor, Chlor oder Brom, insbesondere um Brom oder Chlor;
C₁-C₄-Alkyl bedeutet Methyl, Ethyl, n-Propyl, Isopropyl, n-Butyl, sec.-Butyl, i-Butyl oder tert.- Butyl;
bei C₁-C₆-Alkyl handelt es sich beispielsweise um Methyl, Ethyl, n-Propyl, Isopropyl, n-Butyl, sec.-Butyl, tert.-Butyl, n-Amyl, tert.-Amyl, Hexyl und bei C₁-C₁₈-Alkyl zusätzlich z.B. um Heptyl, Octyl, 2-Ethylhexyl, Nonyl, Decyl, Dodecyl, Tetradecyl, Hexadecyl oder Octadecyl;
C₁-C₆-Alkoxy bedeutet z.B. Methoxy, Ethoxy, n-Propoxy, Isopropoxy, Butyloxy, oder Hexyloxy;
C₁-C₁₈-Alkylmercapto steht beispielsweise für Methylmercapto, Ethylmercapto, Propylmercapto, Butylmercapto oder Hexyl mercapto;
C₁-C₆-Alkylamino bedeutet z.B. Methylamino, Ethylamino, Propylamino oder Hexylamino;
C₅-C₆-Cycloalkyl steht z.B. für Cyclopentyl und insbesondere für Cyclohexyl.

Von besonderem Interesse ist das erfindungsgemässe Verfahren mit Verbindungen der Formeln I, II und III, worin
R₁, R₂ und R₃ unabhängig voneinander Wasserstoff, Chlor, Brom, Methyl, Ethyl, Methoxy, Ethoxy, Methylamino, Ethylamino, -CN, -NO₂, Phenyl oder Phenoxy bedeuten,
X X' ist,
X' -OR₄ oder -N(R₅)(R₆) bedeutet, worin
R₄ Methyl, Ethyl, Cyclohexyl, unsubstituiertes oder durch Chlor, Methyl, Methoxy, Ethoxy, -CN, -NO₂ oder Dimethylamino substituiertes Phenyl,
R₅ und R₆ unabhängig voneinander Wasserstoff, Methyl, Ethyl, Cyclohexyl, unsubstituiertes oder durch Chlor, Methyl, Ethyl, Methoxy, Ethoxy, Dimethylamino oder -NO₂ substituiertes Phenyl bedeuten oder R₅ und R₆ zusammen mit dem N-Atom, an das sie gebunden sind, einen heterocyclischen Ring ausgewählt aus der Gruppe Pyrrolidinyl, Morpholinyl oder Phthalimidyl bilden,
Y 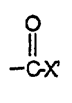 ist und
Z Chlor oder Brom bedeutet.

In den Formeln I und II bedeutet A vorzugsweise eine direkte Bindung, R₁ und R₂ sind bevorzugt identisch und R₃ bedeutet vorzugsweise Chlor, Brom oder C₁-C₄-Alkyl.

Als Alkalimetall- oder Erdalkalimetallformiat kann man Natrium-, Kalium-, Magnesium- und Calciumformiat, bevorzugt Calciumformiat in den für die Verbindungen X'-H der Formel III angegeben Mengen einsetzen. Die Formiate können auch als Basen fungieren.

Verbindungen der Formel II sind bekannt oder nach bekannten Methoden wie in den eingangs genannten US 4,415,685, 4,579,949 und 4 791 204, insbesondere US 4,579,949, beschrieben, herstellbar.

Es wurde gefunden, daß man das Molverhältnis von Diketopyrrolopyrrolen der Formel II zu Ameisensäure oder X'-H in einem großen Bereich variieren kann. Um Verunreinigungen zu vermeiden, empfiehlt sich daher eine Untergrenze von 1:1, bzw. 1:2, falls Y in Formel II -COX bedeutet. Bei Umsetzungen mit Verbindungen X'-H, die ein niedriges Molgewicht aufweisen, wie Methanol, kann das Molverhältnis von Diketopyrrolopyrrolen der Formel II zu X'-H sehr klein werden, insbesondere, wenn die Verbindung X'-H gewünschtenfalls zusätzlich als Lösungsmittel fungieren soll.

In einer bevorzugten Ausführungsform wählt man das Molverhältnis von Diketopyrrolopyrrolen der Formel II zu Ameisensäure oder X'-H oder Alkalimetall- oder Erdalkalimetallformiat im Bereich von 1:1 bis 1:1000, bevorzugt von 1:1 bis 1:20, oder, Erdalkalimetallformiat im Bereich von 1:1 bis 1:1000, bevorzugt von 1:1 bis 1:20, oder, wenn Y in Formel II 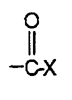 bedeutet, im Bereich von 1:2 bis 1:2000, bevorzugt von 1:4 bis 1:40.

Es wurde ferner gefunden, daß man die Carboxylierung vorteilhaft in einem organischen Lösungsmittel durchführen kann.

Als Lösungsmittel eignen sich üblicherweise alle gängigen, vorzugsweise polaren, organischen Lösungsmittel, aprotisch oder protisch, besonders bevorzugt aprotisch polare, oder Gemische solcher Lösungsmittel untereinander oder mit Wasser, bevorzugt so, dass mindestens eine Komponente polar ist. Zweckmässig sind z.B. N-Methylpyrrolidon, Dimethylformamid oder Dimethylacetamid, bevorzugt N-Methylpyrrolidon.

Geeignete ein- oder mehrzähnige Komplexe von Palladiumkatalysatoren mit tertiären Phosphinen sind in der Regel

Pd [P(C₆H₅)₃]₄ (IV),

PdCl₂[P(C₆H₅)₃]₂ (V),

Pd [P(C₆H₁₁)₃]₄ (VI),

PdCl₂[P(C₆H₁₁)₃]₂ (VII)

und

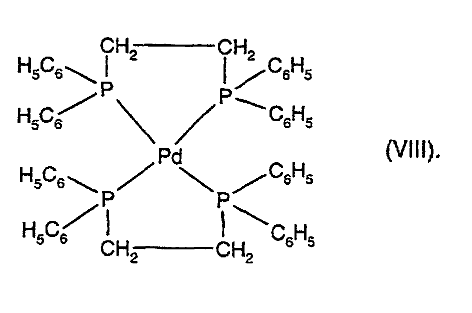

Der Palladiumkatalysator kann und wird bevorzugt auch in situ aus einem geeigneten Palladiumsalz, wie Palladiumchlorid und -acetat, und einem tertiären Phosphin, wie Triphenylphosphin, Tricyclohexylphosphin und Bis(diphenylphosphino)-ethan hergestellt.

Besonders bevorzugt werden Palladium-(II)-chlorid und Triphenylphosphin eingesetzt.

Der Katalysator wird vorzugsweise in einer Menge von 0,01 bis 50, bevorzugt 0,1 bis 30 Mol-%, bezogen auf das Diketopyrrolopyrrol der Formel II zugegeben.

Geeignete Basen sind beispielsweise tertiäre Amine wie Triethylamin, Tripropylamin, Tributylamin, N-Methyl-piperidin, N-Methyl-morpholin oder Benzyldimethylamin, sowie Alkalimetall- oder Erdalkalimetallsalze von Carbonsäuren mit 1-4 C-Atomen, wie Natrium-, Kaliumacetat, Natriumformiat, Kaliumformiat und Calciumformiat, sowie Alkalimetall- oder Erdalkalimetallsalze schwacher Mineralsäuren, wie Kalium- oder Natriumphosphat.

Falls man die Carboxylierung mit einem primären oder einem sekundären Amin der Formel X'-H (III) durchführt, worin X' -N(R₅)(R₆) bedeutet, dann kann auch einen Überschuss dieses Amins als Base dienen. Vorzugsweise setzt man dann 1 bis 20 Mol, bevorzugt 2 bis 10 Mol, eines Überschusses, bezogen auf das eingesetzte Diketopyrrolopyrrol II, oder 1 bis 40 Mol, bevorzugt 4 bis 20 Mol, falls das eingesetzte Diketopyrrolopyrrol II zwei -COX-Gruppen enthält, ein.

Vorzugsweise wird ein tertiäres Amin, insbesondere Triethylamin verwendet.

Die Base wird zweckmässig in einer Menge von 1 bis 20, bevorzugt 1 bis 4 Moläquivalente bezogen auf das Diketopyrrolopyrrol der Formel II, wenn Y R₃ bedeutet, und von 2 bis 40, bevorzugt 2 bis 10 Moläquivalente, wenn Y 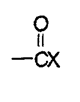 ist, zugegeben.

Das erfindungsgemässe Verfahren wird bevorzugt bei einem Druck zwischen 100 kPa (1 bar) und 10 MPa, vorzugsweise von 100 kPa bis 5 MPa (50 bar), und bei einer Temperatur von 10 bis 220, bevorzugt von 50 und 200, insbesondere bevorzugt von 100 bis 180°C, durchgeführt. Besonders bevorzugt führt man die Reaktion bei einem Kohlenmonoxid-Partialdruck im Bereich von 0,1 bis 5 MPa, insbesondere von 0,5 bis 2,5 MPa durch, falls man die Carboxylierung mit der Verbindung X'-H der Formel III vornimmt.

Die erfindungsgemäss erhaltenen Diketopyrrolopyrrol-Carbonsäuren, -Ester und -Amide besitzen, wie schon aus den eingangs erwähnten US-Patenten 4 415 685, 4 579 949, 4 791 204 sowie 5 476 886 zu entnehmen ist, gute Pigmenteigenschaften und eignen sich demnach sehr gut zum Pigmentieren von hochmolekularem organischem Material. Sie zeichnen sich insbesondere durch hohe Farbtonreinheit aus.

Je nach Anwendung können die erfindungsgemäss hergestellten Verbindungen direkt nach Synthese oder nach einer für Pigmente üblichen und allgemein bekannten Nachbehandlung als Pigmente verwendet werden.

Die nachfolgenden Beispiele erläutern die Erfindung.

Beispiel 1: In einen Autoklaven werden 4,46 g der Verbindung der Formel 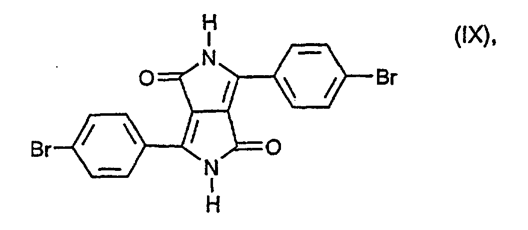 sowie 0,18 g Palladium (II)-chlorid, 1,58 g Triphenylphosphin, 6 ml Triethylamin, 10 ml Methanol und 180 ml N-Methylpyrrolidon gefüllt. Nach Verdrängen der Luft durch Stickstoff werden 10 bar Kohlenmonoxid aufgepresst und unter Rühren während 24 Stunden auf 120°C geheizt.

Die erhaltene Suspension wird auf 1 l Wasser gegossen, der Niederschlag abfiltriert und nacheinander mit Wasser, mit Methanol und mit Methyl-tert-butylether gewaschen und dann bei 60°C im Vakuum getrocknet. Man erhält 3,4 g (84 %) des Pigmentes der Formel

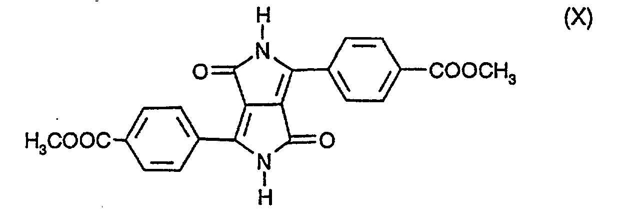

Beispiel 2: In einen Autoklaven werden 4,46 g der Verbindung der Formel IX sowie 0,18 g Palladium(II)-chlorid, 1,58 g Triphenylphosphin, 10 ml n-Butylamin und 180 ml N-Methylpyrrolidon gefüllt. Nach Verdrängen der Luft durch Stickstoff werden 10 bar Kohlenmonoxid aufgepresst und unter Rühren während 24 Stunden auf 120°C geheizt.

Die erhaltene Suspension wird auf 1 l Wasser gegossen, der Niederschlag abfiltriert und nacheinander mit Wasser, mit Methanol und mit Methyl-tert-butylether gewaschen und dann bei 60°C im Vakuum getrocknet. Man erhält 4,24 g (87 %) des Pigmentes der Formel

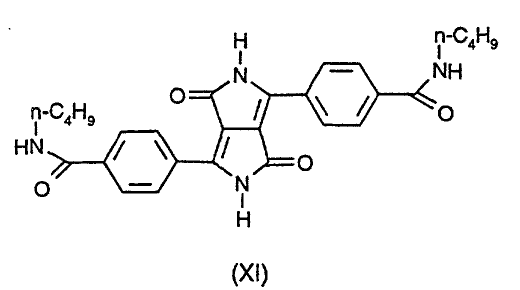

Beispiel 3: In einen Autoklaven werden 4,46 g der Verbindung der Formel IX sowie 0,18 g Palladium(II)-chlorid, 1,58 g Triphenylphosphin, 6 ml Triethylamin, 4 ml Anilin und 250 ml N-Methylpyrrolidon gefüllt. Nach Verdrängen der Luft durch Stickstoff werden 10 bar Kohlenmonoxid aufgepresst und unter Rühren während 40 Stunden auf 120°C geheizt.

Die erhaltene Suspension wird auf 1 l Wasser gegossen, der Niederschlag abfiltriert und nacheinander mit Wasser, mit Methanol und mit Methyl-tert-butylether gewaschen und dann bei 60°C im Vakuum getrocknet. Man erhält 4,65 (88 %) des Pigmentes der Formel

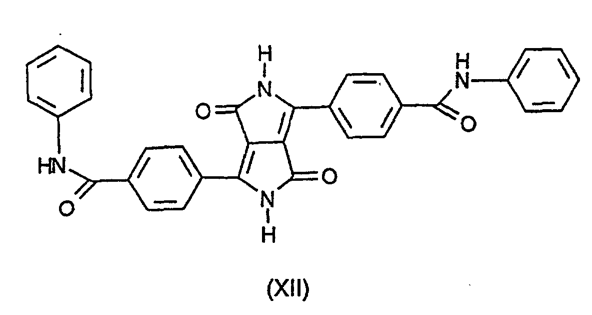

Beispiel 4: In einen Autoklaven werden 4,46 g der Verbindung der Formel IX sowie 0,18 g Palladium(ll)-chlorid, 1,58 g Triphenylphosphin, 5,0 g 2-Aminoethansulfonsäure, 15 ml Triethylamin und 180 ml N-Methylpyrrolidon gefüllt. Nach Verdrängen der Luft durch Stickstoff werden 10 bar Kohlenmonoxid aufgepresst und unter Rühren während 24 Stunden auf 120°C geheizt.

Die erhaltene Suspension wird auf eine Lösung von 8 ml konz. Schwefelsäure in 500 ml Wasser gegossen, die entstandene Lösung auf 60°C erwärmt und mit 160 g NaCI versetzt. Der Niederschlag wird abfiltriert und zunächst mit gesättigter wässriger NaCI-Lösung säurefrei gewaschen, dann mit Wasser solange "salzfrei" gewaschen, bis das Filtrat beginnt sich rot zu verfärben. Nach Trocknen bei 80°C im Vakuum erhält man 4,93 g (78 %) des Pigmentes der Formel

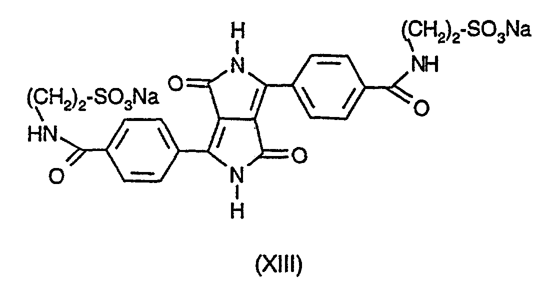

Beispiel 5: In einen Autoklaven werden 4,46 g der para-Dibrom-Verbindung (IX) (siehe Beispiel 1) sowie 0,18 g Palladium(ll)-chlorid, 1,58 g Triphenylphosphin, 1,56 g Calciumformiat und 200 ml N-Methylpyrrolidon gefüllt. Nach Verdrängen der Luft durch Stickstoff werden 5 bar Kohlenmonoxid aufgepresst und unter Rühren während 24 Stunden auf 120°C geheizt.

Die erhaltene Suspension wird filtriert, der Rückstand mit 200 ml Wasser, dann mit 200 ml 5%iger wäßriger Schwefelsäure und dann mit Wasser bis zum neutralen Ablauf gewaschen und bei 60°C im Vakuum getrocknet. Das Filtrat wird auf 250 ml Wasser gegossen, der ausfallende Niederschlag mit 200 ml 5%-iger wässriger Schwefelsäure und dann mit Wasser bis zum neutralen Ablauf gewaschen und bei 60°C im Vakuum getrocknet.

Die erhaltenen vereinigten Produkte werden mit 50 ml Methyl-tert.-butylether extrahiert und der Rückstand bei 70°C im Vakuum getrocknet.

Man erhält 3.5 g (93 %) des Pigmentes der Formel

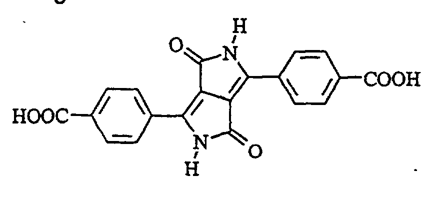

Beispiel 6: In einen Autoklaven werden 8,92 g der p-Dibromverbindung der Formel (IX) sowie 0,36 g Palladium(II)-chlorid, 3,16 g Triphenylphosphin, 5,44 g Imidazol, 12 ml Triethylamin und 320 ml N-Methylpyrrolidon gefüllt. Nach Verdrängen der Luft durch Stichstoff werden 10 bar Kohlenmonoxid aufgepresst und unter Rühren während 24 Stunden auf 120°C geheizt.

Die erhaltene Suspension wird auf 1 l Toluol gegossen, der Niederschlag abfiltriert und während 1 Stunde mit 130 ml Methylenchlorid gerührt. Nach erneuter Filtration wird mit 50 ml Ether gewaschen und dann bei 60°C im Vakuum getrocknet.

Man erhält 7,0 g (74 %) des Pigmentes der Formel

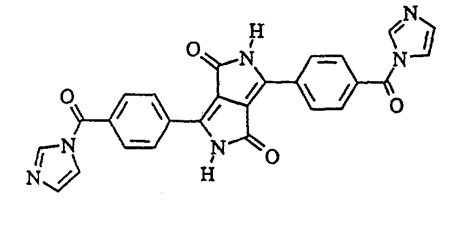

## Patentansprüche

1. Verfahren zur Herstellung von Diketopyrrolopyrrolen der Formel 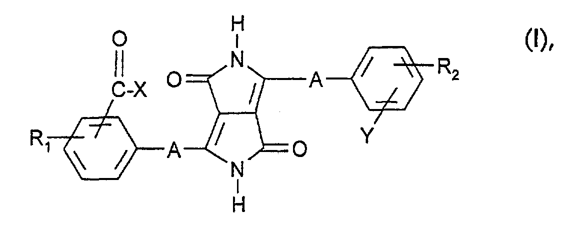 durch Umsetzung eines Diketopyrrolopyrrols der Formel 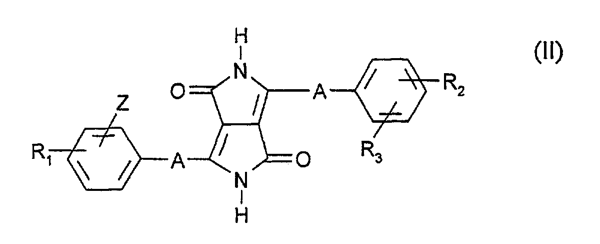 mit
a) Ameisensäure oder
b) einer Verbindung der Formel
X'-H (III)
oder einem Alkalimetall- oder einem Erdalkalimetallformiat
zusammen mit Kohlenmonoxid
in Gegenwart eines ein- oder mehrzähnigen Palladiumkatalysators, der tertiäre Phosphine als Liganden enthält, und einer Base,
wobei in den Formeln I, II und III
A eine direkte Bindung oder eine Gruppe 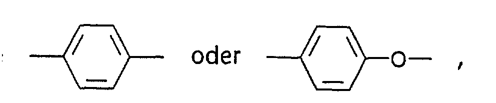 wobei das Phenylen an das Pyrrolopyrrol gebunden ist, bedeutet,
R₁, R₂ und R₃ unabhängig voneinander Wasserstoff, Halogen, C₁-C₆-Alkyl, C₅-C₆-Cycloalkyl, C₁-C₆-Alkoxy, C₁-C₆-Alkylamino, C₁-C₆-Alkylmercapto, -CF₃, -CN order -NO₂ bedeuten,
X X' oder OH ist,
X' -OR₄ oder -N(R₅)(R₆) bedeutet, worin
R₄ C₁-C₁₈-Alkyl, C₅-C₆-Cycloalkyl, unsubstituiertes oder durch Halogen, C₁-C₆-Alkyl, C₁-C₆-Alkoxy, C₁-C₆-Alkylamino, -CN oder -NO₂ substituiertes Phenyl oder Naphthyl ist,
R₅ und R₆ unabhängig voneinander Wasserstoff, C₁-C₁₈-Alkyl, C₅-C₆-Cycloalkyl, unsubstituiertes oder durch Halogen, C₁-C₆-Alkyl, C₁-C₆-Alkoxy, C₄-C₆-Alkylamino, -CN oder -NO₂ substituiertes Phenyl oder Naphthyl oder eine Gruppe -(CH₂)ₙ-SO₃R₇ bedeuten, worin R₇ Wasserstoff, Kalium oder Natrium und n eine ganze Zahl zwischen 2 und 6 sind, oder R₅ und R₆ zusammen mit dem N-Atom, an das sie gebunden sind, einen unsubstituierten oder durch C₁-C₆-Alkyl oder Phenyl substituierten 5- oder 6-gliedrigen heterocyclischen Rest ausgewählt aus der Gruppe Pyrrolidinyl, Piperidyl, Pyrrolyl, Triazolyl, Imidazolyl, Pyrazolyl, Piperazinyl, Morpholinyl, Thiomorpholinyl, Phthalimidyl, Carbazolyl, Indolyl, Indazolyl, Benzimidazolyl und Tetrahydrochinolinyl bilden,
Y R₃ oder 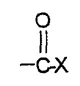 bedeutet, und
Z Halogen ist.

2. Verfahren zur Herstellung von Diketopyrrolopyrrolen der Formel I nach den Anspruch 1, **dadurch gekennzeichnet, daß** man die Reaktion in einem organischen Lösungsmittel durchführt.

3. Verfahren zur Herstellung von Diketopyrrolopyrrolen der Formel I nach den Ansprüchen 1 bis 2, **dadurch gekennzeichnet, daß** man das Molverhältnis von Diketopyrrolopyrrolen der Formel II zu Ameisensäure oder X'-H oder Alkalimetall- oder Erdalkalimetallformiat im Bereich von 1:1 bis 1:1000, oder, wenn Y in Formel II 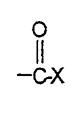 bedeutet, im Bereich von 1:2 bis 1:2000 wählt.

4. Verfahren gemäss den Ansprüchen 1 bis 3, **dadurch gekennzeichnet, daß** man, falls X' eine Aminogruppe -N(R₅)(R₆) bedeutet, als Base einen Überschuss an Verbindung der Formel III einsetzt.

5. Verfahren gemäss den Ansprüchen 1 bis 4, **dadurch gekennzeichnet, daß** man die Reaktion bei einem Kohlenmonoxid-Partialdruck im Bereich von 0,1 bis 5 MPa durchführt.

## Claims

1. A process for the preparation of a diketopyrrolopyrrole of formula 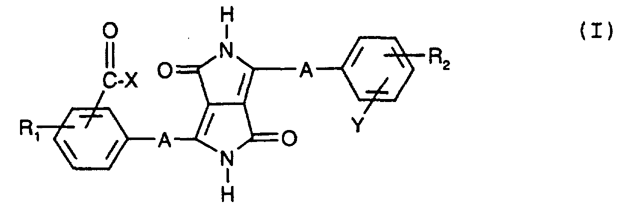 by reacting a diketopyrrolopyrrole of formula 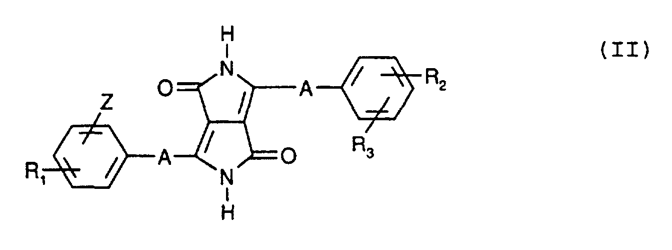 with
a) formic acid or
b) a compound of formula III
X'-H (III)
or with an alkali metal formate or alkaline earth metal formate
together with carbon monoxide
in the presence of a unidentate or polydentate palladium catalyst containing tertiary phosphine ligands, and a base,
where, in formulae I, II and III,
A is a direct bond or a group 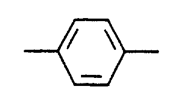 or 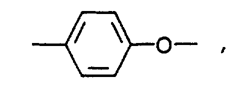 where the phenylene is attached to the pyrrolopyrrole,
R₁, R₂ and R₃ are each independently of one another hydrogen, halogen, C₁-C₆alkyl, C₅-C₆-cycloalkyl, C₁-C₆alkoxy, C₁-C₆alkylamino, C₁-C₆alkylmercapto, -CF₃, -CN or -NO₂,
X is X' or OH,
X' is -OR₄ or -N(R₅) (R₆), in which
R₄ is C₁-C₁₈alkyl, C₅-C₆cycloalkyl; phenyl or naphthyl which is unsubstituted or substituted by halogen, C₁-C₆alkyl, C₁-C₆alkoxy, C₁-C₆alkylamino, -CN or -NO₂,
R₅ and R₆ are each independently of the other hydrogen, C₁-C₁₈alkyl, C₅-C₆cycloalkyl; phenyl or naphthyl which is unsubstituted or substituted by halogen, C₁-C₆alkyl, C₁-C₆-alkoxy, C₄-C₆alkylamino, -CN or -NO₂, or a group -(CH₂)ₙ-SO₃R₇, in which R₇ is hydrogen, potassium or sodium and n is an integer from 2 to 6, or R₅ and R₆, together with the nitrogen atom to which they are attached; are an unsubstituted or C₁-C₆alkyl- or phenyl-substituted 5- or 6-membered heterocyclic radical selected from the group consisting of pyrrolidinyl, piperidyl, pyrrolyl, triazolyl, imidazolyl, pyrazolyl, piperazinyl, morpholinyl, thiomorpholinyl, phthalimidyl, carbazolyl, indolyl, indazolyl, benzimidazolyl and tetrahydroquinolinyl,
Y is R₃ or 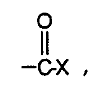 and
Z is halogen.

2. A process for the preparation of a diketopyrrolopyrrole of formula I according to claim 1, which comprises carrying out the reaction in an organic solvent.

3. A process for the preparation of a diketopyrrolopyrrole of formula I according to claims 1 to 2, which comprises choosing the molar ratio of diketopyrrolo-pyrroles of formula II to formic acid or X'-H or to alkali metal formate or alkaline earth metal formate in the range from 1:1 to 1:1000 or, if Y in formula II is 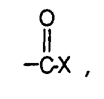 in the range from 1:2 to 1:2000.

4. A process according to claims 1 to 3, wherein, if X' is an amino group -N(R₅)(R₆), an excess of compound of formula III is used as base.

5. A process according to claims 1 to 4, which comprises carrying out the reaction at a carbon monoxide partial pressure in the range from 0.1 to 5 MPa.

## Revendications

1. Procédé pour la préparation de dicétopyrrolopyrroles de formule 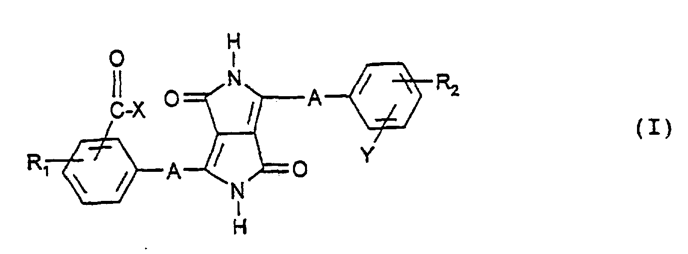 par réaction d'un dicétopyrrolopyrrole de formule 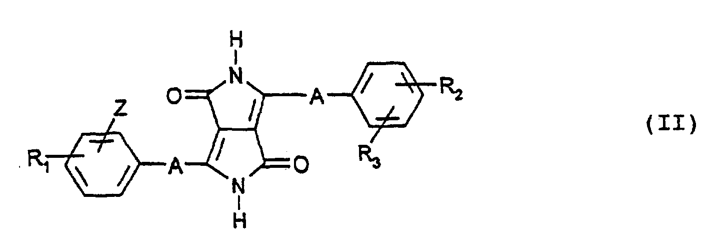 avec
a) l'acide formique ou
b) un composé de formule
X'-H (III)
ou avec un formiate de métal alcalin ou un formiate de métal alcalino-terreux conjointement avec le monoxyde de carbone
en présence d'un catalyseur au palladium mono- ou multidenté qui contient des phosphines comme ligands tertiaires, et avec une base
où dans les formules I, II et III
A représente une liaison directe ou un groupe 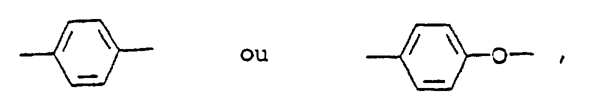 le groupe phénylène est lié au pyrrolopyrrole,
R₁, R₂ et R₃ représentent, indépendamment les uns des autres, des atomes d'hydrogène, des atomes d'halogène, des groupes alkyle en C₁-C₆, cycloalkyle en C₅-C₆, alkoxy en C₁-C₆, (alkyl en C₁-C₆)amino, (alkyl en C₁-C₆)mercapto, -CF₃, -CN ou -NO₂,
X représente X' ou un groupe OH,
X' représente des groupes -OR₄ ou -N(R₅)(R₆), où
R₄ représente des groupes alkyle en C₁-C₁₈, cycloalkyle en C₅-C₆, phényle ou naphtyle non substitué ou substitué par des substituants halogène, alkyle en C₁-C₆, alkoxy en C₁-C₆, (alkyl en C₁-C₆)amino, -CN ou -NO₂,
R₅ et R₆ représentent, indépendamment l'un de l'autre, des atomes d'hydrogène, des groupes alkyle en C₁-C₁₈, cycloalkyle en C₅-C₆, phényle ou naphtyle non substitué ou substitué par des substituants halogène, alkyle en C₁-C₆, alkoxy en C₁-C₆, (alkyl en C₁-C₆)amino, -CN ou -NO₂, ou représentent un groupe -(CH₂)ₙ-SO₃R₇, où R₇ représente un atome d'hydrogène, un atome de potassium ou de sodium et n est un entier compris entre 2 et 6, ou R₅ et R₆ forment, ensemble avec l'atome de N auquel ils sont liés, un reste hétérocyclique à 5 ou 6 chaînons non substitué ou substitué par des substituants alkyle en C₁-C₆ ou phényle, pris dans le groupe comportant pyrrolidinyle, pipéridyle, pyrrolyle, triazolyle, imidazolyle, pyrazolyle, pipérazinyle, morpholinyle, thiomorpholinyle, phtalimidyle, carbazolyle, indolyle, indazolyle, benzimidazolyle et tétraquinoléinyle,
Y représente R₃ ou un groupe 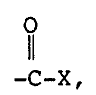 et
Z représente un atome d'halogène.

2. Procédé pour la préparation de dicétopyrrolopyrroles de formule I selon la revendication 1, **caractérisé en ce qu'**on met en oeuvre la réaction dans un solvant organique.

3. Procédé pour la préparation de dicétopyrrolopyrroles de formule I selon les revendications 1 à 2, **caractérisé en ce qu'**on choisit le rapport molaire des dicétopyrrolopyrroles de formule II à l'acide formique ou à X'-H ou au formiate de métaux alcalins ou alcalino-terreux dans le domaine de 1:1 à 1:1000 ou. lorsque Y dans la formule II représente un groupe 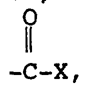 dans le domaine de 1:2 à 1:2000.

4. Procédé selon les revendications 1 à 3, **caractérisé en ce qu'**on utilise, dans le cas où X' représente un groupe amino -N(R₅)(R₆), en tant que base, un excès en composé de formule III.

5. Procédé selon les revendications 1 à 4, **caractérisé en ce qu'**on met en oeuvre la réaction à une pression partielle de monoxyde de carbone dans le domaine de 0,1 à 5 MPa.
